# FASCICULE DE BREVET EUROPEEN

(11) **EP 0 301 943 B1**
(45) Date de publication et mention de la délivrance du brevet: **12.05.1993**
(21) Numéro de dépôt: 88401878.9
(22) Date de dépôt: 20.07.1988
(51) Int. Cl.: A61K 35/78, A61K 9/50

(54) **Produit pharmaceutique laxatif**
Pharmazeutisches Laxiermittel
Pharmaceutical laxative

(30) Priorité: 22.07.1987 FR 8710370
(43) Date de publication de la demande: 01.02.1989
(73) Titulaire: SOMALEAD S.A., F-75017 Paris (FR)
(72) Inventeur: Bourquin, Camille, F-94140 Alforville (FR); Vendel, Jean-René, F-92100 Boulogne (FR)
(74) Mandataire: Laget, Jean-Loup

(56) Documents cités:
- US-A- 2 111 286
- US-A- 2 800 458

## Description

L'invention concerne un produit pharmaceutique laxatif.

On distingue en général deux types de produits laxatifs : les laxatifs lubrifiants, par exemple à base de paraffine liquide ; et les laxatifs de lest, notamment d'origine végétale, à base de substances mucilagineuses.

Comme laxatif lubrifiant, le document US A 2.111.286 décrit une combinaison de fibres de kapok et de lubrifiant minéral, dans laquelle les fibres de kapok n'ont qu'un rôle physique de véhicule ou de support du lubrifiant minéral.

Il est par ailleurs connu d'encapsuler des huiles minérales.

Le document US A 2.800.458 décrit un procédé de fabrication de micro-capsules contenant notamment des huiles minérales utilisées comme solvants de produits liposolubles tels que des encres. Les micro-capsules sont utilisées dans la composition de films de transfert pour la confection de papier autocopiant.

Les laxatifs lubrifiants présentent un inconvénient : ils sont fréquemment générateurs de suintement anal. Les laxatifs de lest présentent un autre inconvénient : ils aggravent en général, et surtout en début de traitement, les ballonnements qui accompagnent habituellement la constipation.

Un but de la présente invention est de proposer un laxatif qui ne présente ni l'inconvénient des laxatifs lubrifiants à base de paraffine liquide, ni l'inconvénient des laxatifs de lest d'origine végétale.

L'invention a pour objet un produit pharmaceutique laxatif du type comportant un laxatif lubrifiant minéral et un laxatif de lest d'origine végétale, caractérisé en ce que le laxatif lubrifiant est une paraffine liquide microencapsulée dans la gélatine et se présente sous forme sèche, et en ce que le laxatif de lest est de la poudre d'Ispaghul obtenue par décortication de graines de psyllium et réduction en poudre de l'enveloppe des graines ; les proportions des constituants étant les suivantes : 25 a 40% de paraffine liquide microencapsulée, 25 a 40% de poudre d'Ispaghul, le reste étant constitué par un excipient.

Le produit pharmaceutique laxatif selon l'invention est une association médicamenteuse de paraffine liquide microencapsulée et de poudre d'Ispaghul. Il se présente sous forme d'une poudre, éventuellement aromatisée, que l'on disperse dans un grand verre d'eau ou de jus de fruits, avant de l'ingérer.

La paraffine liquide, microencapsulée dans la gélatine, se présente sous une forme originale sèche.

La poudre d'Ispaghul est obtenue par décortication de graines de psyllium (plantago ovata Forsk). Le tégument recueilli, qui correspond à l'enveloppe des graines, est réduit en poudre. Il contient des susbtances mucilagineuses qui confèrent à la poudre ses propriétés de gonflement dans l'eau.

Les deux constituants principaux du produit pharmaceutique selon l'invention sont présents dans les proportions suivantes :
25 à 40% de paraffine liquide microencapsulée
25 à 40% de poudre d' Ispaghul
le reste étant constitué par un excipient
contenant par exemple du sorbitol,
de la cellulose microcristalline et des arômes. L'étude de la toxicité du produit selon l'invention a été réalisée bien que ses constituants ne semblent pas susceptibles de faire apparaître une toxicité singulière. Elle comporte une étude, chez le rat et la souris, de la toxicité aiguë de chaque constituant et du produit complet, et une étude, chez le rat et le lapin, de la toxicité à terme du produit complet aux doses de 2.500 et 5.000 mg/kg pendant 30 jours. Les rares anomalies observées au cours de cette étude sont dues à des phénomènes d'encombrement liés à la consistance des préparations administrées. L'étude démontre l'absence d'une toxicité intrinsèque du produit.

Le produit pharmaceutique laxatif selon l'invention a été administré, dans un essai clinique, à 93 sujets souffrant de constipation chronique idiopathique. 45 sujets ont reçu le produit en ouvert, et 48 en double-insu contrôlé. L'efficacité laxative du produit a été constatée sur la base de l'augmentation du nombre de selles par semaine, et de l'amélioration du caractère des selles. En outre, le produit présente deux avantages par rapport aux laxatifs connus, en ce qui concerne la tolérance clinique.

Tout d'abord, les préparations laxatives à base de substances mucilagineuses aggravent en général et surtout en début de traitement, les ballonnements qui accompagnent habituellement la constipation. Le produit selon l'invention semble, au contraire, faire régresser ce genre d'effet indésirable.

Ensuite, les traitements laxatifs à base de paraffine liquide sont fréquemment générateurs de suintement anal. Avec la forme sèche microencapsulée de paraffine liquide, aucun cas de cet effet secondaire gênant n'a été relevé, au cours de l'essai clinique.

## Revendications

1. Produit pharmaceutique laxatif du type comportant un laxatif lubrifiant minéral et un laxatif de lest d'origine végétale, caractérisé en ce que le laxatif lubrifiant est une paraffine liquide microencapsulée dans la gélatine et se présente sous forme sèche, et en ce que le laxatif de lest est de la poudre d'Ispaghul obtenue par décortication de graines de psyllium et réduction en poudre de l'enveloppe des graines ; les proportions des constituants étant les suivantes : 25 à 40% de paraffine liquide microencapsulée, 25 à 40% de poudre d'Ispaghul, le reste étant constitué par un excipient.

## Claims

1. A pharmaceutical laxative product of the type comprising a mineral lubricant laxative and a ballast laxative of vegetable origin, characterised in that the lubricant laxative is a liquid paraffin microencapsulated in gelatin and presented in a dry form, and in that the ballast laxative is Ispaghul powder obtained from hulling grains of psyllium and reducing the grain envelopes into powder; the proportions of the constituents being as follows : 25 to 40% of microencapsulated liquid paraffin, 25 to 40% of Ispaghul powder, the rest being made up by an excipient.

## Patentansprüche

1. Pharmazeutisches Laxiermittel, von der ein minerales schmierendes Abführmittel und ein aus pflanzlicher Herkunft als Ballaststoff dienende Abführmittel enthaltender Art, dadurch gekennzeichnet, daß das schmierende Abführmittel aus einem in Gelatinemikrokapseln präparierten flüssigen Paraffin besteht und in trockener Form präsentiert ist, und daß das als Ballaststoff dienende Abführmittel ein nach Enthülsen von Psylliumkörnern und Pulverisieren der Hülse dieser Körner gewonnenes Ispaghul Pulver ist ; mit folgenden Bestandteilgehalten : 25 bis 40% von in Mikrokapseln präpariertem flüssigem Paraffin, 25 bis 40% von Ispaghul Pulver, der überrest bestehend aus einer Grundmasse.
